Europäisches Patentamt

European Patent Office

Office européen des brevets

(11) Publication number: **0 138 262**
**B1**

(12) **EUROPEAN PATENT SPECIFICATION**

(45) Date of publication of patent specification: **13.06.90**

(51) Int. Cl.⁵: **A 61 K 7/42**

(21) Application number: **84201374.0**

(22) Date of filing: **25.09.84**

(54) Composition for treatment of skin affections and its application.

(30) Priority: **26.09.83 NL 8303283**

(43) Date of publication of application:
**24.04.85 Bulletin 85/17**

(45) Publication of the grant of the patent:
**13.06.90 Bulletin 90/24**

(84) Designated Contracting States:
**AT BE CH DE FR GB IT LI LU NL SE**

(56) References cited:
**DE-A-2 651 617      FR-A-2 431 863**
**DE-B-1 095 463      GB-A- 864 313**
**FR-A- 929 821       GB-A-1 201 014**
**FR-A-2 209 561**

**Chemical Abstracts, vol. 97, no. 19, November 8,
1982, page 339, ref. no. 158809h; Columbus,
Ohio, US Setsuko Ohta et al.:"Studies on
chemical protectors against radiation.
XXII.Protective effect of various sulfur
containing compounds on skin injury induced
by x-irradiation"**

(73) Proprietor: **Chemisch Adviesbureau Drs. J.C.P.
Schreuder B.V.**
**P.O. Box 430**
**NL-3740 AK Baarn (NL)**

(72) Inventor: **Schreuder, J.C.P., Drs.**
**Postbus 430**
**NL-3740 AK Baarn (NL)**

(74) Representative: **Hiltl, Elmar, Dr. et al
KOHLER, GLAESER, HILTL Patentanwälte
Flüggenstrasse 13
D-8000 München 19 (DE)**

Courier Press, Leamington Spa, England.

# Description

The invention is relating to a composition for the treatment of skin affections and more particularly to a composition for treatment of so called sun eczema or dishydrosis.

Sun-eczema or dishydrosis usually manifests by means of sun vesicles, skin irritations and itch.

Moreover the invention is relating to the application of such a composition for the fight against of the beforementioned symptoms. Already several means and compositions were proposed in the past for this purpose, such as compositions consisting of vaseline (paraffins), salicylic acid and benzoic acid or an alcoholic solution of resorcine (0,001% by weight).

However, with these formerly proposed means a quick and adequate fight against the beforementioned symptoms could not be attained. On the one hand this may be attributed to the fact, that these formerly proposed compositions usually were containing fat or fatty compounds, which made return the apparently disappeared symptoms in a much worse degree after a necessarily rather long use of these means.

On the other hand the more and more often exposure of the skin to the radiation of sun, solaria, etc. which is regarded to a major degree responsible for this sun affections seems to give rise to such symptoms in still more extensive degree, whereby moreover the skin is usually treated, preceding such a radiation, with oil or fat containing creams for the promotion of the desired browning of the skin and/or for the protection against the action of specific radiation, which accompanies the desired one.

Therefore there is a still growing need for compositions for an efficient and quick treatment of this sun eczema or dishydrosis.

Surprisingly there could be found as result of extensive research and experimentation an adequate composition, which comprises at least the following ingredients:

panthenol, in an amount of from 0.1 to 5% by weight, calculated on the weight of the total composition and preferably in an amount of from 1—2% by weight

methionine, in an amount of from 0.1 to 3% by weight calculated on the weight of the total composition and preferably in an amount of 1—2% by weight, or

cystine, in an amount of at most 0.009% by weight, or a derivative of cysteine, obtained by acylation of the amino group or the sulph-hydryl-group such as N-acetylcysteine,S-acetylcysteine in an amount of from 0.1 to 3% by weight and more preferably 1—2% by weight. Methionine is preferably applied for the most attractive results.

It will be appreciated by a person skilled in the art that instead of the addition of methionine or cystine as such to the composition, this component may also be formed in situ from the therefore suitable means, which provide this component by a spontaneous conversion.

carraghenate, in an amount of from 0.25 to 4% by weight, calculated on the weight of the total composition and preferably in an amount of from 1—2% by weight.

The meant carraghenate is preferably consisting of a polysaccharide, bearing sulfonic acid residues, being of a natural origin, such as those derived from seaweed. The sulfonic acid residues optionally may have been esterified by glycol, propylene glycol and glycerine (so called modified carraghenates). Such carraghenates experimentally appeared to effect a surprisingly attractive stabilizing effect of the total composition to be applied on the skin, while as an additional advantageous effect, the known attractive properties of such carraghenates, such as elimination of an eventual hardening of sore tissue and the herewith connected curing without or with less extensive scars, as well as the advantageous healing effect and the complex forming properties with proteins, appeared to be maintained in the final total system. The hereinbefore mentioned amounts of carraghenate cause in the final composition the gel structure with a viscosity of from 1000—5000 mPa.s (centipoises), which is desired for and adequate application. It will be appreciated by a person skilled in the art of this specific area, that the carraghenate possibly may partially be replaced by alternative gel forming means such as carboxymethylcellulose, esterified polyacrylic acid (such as Carbopol®), hydroxy ethyl cellulose, in an amount which leads to a viscosity of the final composition in the same desired, hereinbefore mentioned specified range.

sodiumchloride, in an amount of from 0.1 to 5% by weight, calculated on the weight of the total composition and preferably of 0.9% by weight.

a preservative, such as an ester of parahydroxy benzoic acid and preferably the methyl and/or the propyl ester, in an amount of from 0.1—2% by weight, calculated on the weight of the total composition and preferably in an amount of from 0.3.—1% by weight.

Preferably an amount of 0.1% by weight of the propyl ester and 0.2% by weight of the methyl ester is applied.

The hereinbefore mentioned preservatives may be replaced in total or partially by phenyl glycol.

Preferably mixtures of methyl- and/or propyl- and/or butyl-p-hydroxybenzoate and phenylglycol (Phenonip®) are applied.

water ad 100% by weight, calculated on the weight of the total composition.

In addition to the hereinbefore mentioned primary basic ingredients, one or more secondary ingredients may be added if desired, such as perfume in an amount of from 0.1—0.5% by weight, calculated on the weight of the total composition and preferably in an amount of 0.2% by weight and/or antioxydants in an amount of from 0.01—0.3% by weight, calculated on the weight of the total composition and preferably in an amount of 0.1% by weight.

The pH of the total composition is adjusted to a value from 5—6 and preferably to the normal pH of the outer skinlayers of from 5.5—5.8.

As to the earlier proposed compositions the present composition shows a fast and efficient action, accompanied with a fast decrease of the itch and shows a softening activity on the irritated skin.

The compositions according to the present invention may be prepared by methods usually applied for similar compositions.

Preferably there is started with the aqueous phase, in which panthenol, methionine and/or cystine or a derivative of cysteine, sodium chloride and Phenonip® or other preservatives are dissolved followed by addition and dissolution of the carraghenate.

It will be appreciated by a person skilled in the art that the treatment of the beforementioned skin-affections with the present compositions has to be regarded as a feature of the invention too.

Such a treatment preferably comprises a process, whereby an amount of from 1—6 ml of the composition is carefully applied on the surface of the skin to be treated and is spread evenly, after thorough and careful cleaning of the skin with water and soap and/or an alcoholic solution for the removal of oily or fatty compounds.

This treatment has preferably to be carried out 3—6 times a day.

The invention is illustrated on basis of the subsequent examples:

Example 1
A composition composed of:

| | |
|---|---|
| Distilled water | ad 500 g. |
| Panthenol | 5 g. |
| Methionine | 5 g. |
| Carraghenate (Aubigum X₂®) | 10 g. |
| Sodium chloride | 4 g. |
| Preservative (Phenonip®) | 3 g. |
| Antioxydant | 0.05 g. |
| Perfume solution | 1 g. |

is prepared by mixing together of the panthenol, methionine, sodium chloride and Phenonip®, antioxydant and the perfume solution into the aqueous phase, until a solution is obtained whereafter the carraghenate is added under stirring until its complete dissolution.

Example 2
A similar composition was prepared in the same way, while instead of methionine, cystine was added in an amount of 0,045 g. and instead of Phenonip® 0.5 g. of propyl-p-hydroxybenzoate was used, and 1 g. of methyl-p-hydroxybenzoate.

Example 3
In the same way as described in example 1, a composition was prepared, composed of:

| | |
|---|---|
| Distilled water | ad 100% by weight |
| Panthenol | 0.5% |
| Methionine | 1% |
| Sodium chloride | 0.90% |
| Preservative (Phenonip®) | 0.75% |
| Carraghenate (Aubigum X₂®) | 2% |

Example 4
In the same way as described in example 1, a composition was prepared, composed of:

| | |
|---|---|
| Distilled water | ad 100% by weight |
| Panthenol | 1% |
| Methionine | 1% |
| Cystine | 0,009% |
| Sodium chloride | 0,9% |
| Methyl-p-hydroxybenzoate | 0,2% |
| Propyl-p-hydroxybenzoate | 0,1% |
| Carraghenate | 1,5% |

Example 5
In the same way as described in example 1, a composition was prepared, composed of:

| | |
|---|---|
| Distilled water | ad 100% by weight |
| Panthenol | 1% |
| Methionine | 2% |
| Sodium chloride | 0.5% |
| Phenonip® | 1% |
| Carraghenate | 1.5% |

Example 6
In the same way as described in Example 1, a composition was prepared, composed of:

| | |
|---|---|
| Distilled water | ad 100% by weight |
| Panthenol | 2% |
| Methionine | 1% |
| Sodium chloride | 1.2% |
| Phenonip® | 0.4% |
| Methyl-p-hydroxybenzoate | 0.1% |
| Propyl-p-hydroxybenzoate | 0.1% |
| Carraghenate | 1.2% |

Example 7
In the same way as described in example 1, a composition was prepared, composed of:

| | |
|---|---|
| Distilled water | ad 100% by weight |
| Panthenol | 1% |
| Methionine | 1% |
| Sodium chloride | 1% |
| Phenonip® | 0.6% |
| Antioxydant | 0.01% |
| Perfume solution | 0.2% |
| Carraghenate | 1.5% |

**Claims for the Contracting States: BE CH DE FR GB IT LI LU NL SE**

1. Composition for treatment of sun eczema or dishydrosis characterized in that it is at least composed of:

panthenol, in an amount of from 0.1 to 5% by weight, calculated on the weight of the total composition,

methionine, in an amount of from 0.1 to 3% by weight, calculated on the weight of the total composition, or cystine, in an amount of at most 0.009% by weight, calculated on the weight of the total composition, or a derivative of cysteine, obtained by acylation of the aminogroup or acylation of the sulphydrylgroup such as N-acetyl cysteine or S-acetylcysteine in an amount of from 0.1—3% and preferably 1—2% by weight, optionally formed in situ from means providing such compounds due to spontaneous conversion,

carraghenate, in an amount of from 0.25 to 4% by weight, calculated on the weight of the total composition,

sodium chloride, in an amount of from 0.1 to 5% by weight, calculated on the weight of the total composition,

a preservative, in an amount of from 0.1 to 2% by weight, calculated on the weight of the total composition,

water, ad 100% by weight, calculated on the weight of the total composition.

2. Composition according to Claim 1, characterized in that it contains at least panthenol in an amount of from 1 to 2% by weight, calculated on the weight of the total composition.

3. Composition according to Claim 1, characterized in that it contains at least methionine in an amount of from 1 to 2% by weight, calculated on the weight of the total composition.

4. Composition according to Claim 1, characterized in that it contains at least carraghenate in an amount of from 1 to 2% by weight, calculated on the weight of the total composition.

5. Composition according to Claim 1, characterized in that it contains at least sodium chloride in an amount of 0.9% by weight, calculated on the weight of the total composition.

6. Composition according to Claim 1, characterized in that it contains as preservative a methyl- and/or propyl ester of p-hydroxybenzoic acid optionally mixed with phenylglycol.

7. Composition according to Claim 6, characterized in that it contains methyl-p-hydroxybenzoate in an amount of 0.2% by weight, calculated on the weight of the total composition.

8. Composition according to Claim 6, characterized in that it contains propyl-p-hydroxybenzoate in an amount of 0.1% by weight, calculated on the weight of the total composition.

9. Composition according to Claim 1, characterized in that it contains perfume in an amount of from 0.1 to 0.5% by weight, calculated on the weight of the total composition and/or antioxydants in an amount of from 0.01 to 0.3% by weight, calculated on the weight of the total composition.

**Claims for the Contracting State: AT**

1. Process for the preparation of a composition for treatment of sun eczema or dishydrosis characterized in that a composition is prepared by mixing together at least the following ingredients:

panthenol, in an amount of from 0.1 to 5% by weight, calculated on the weight of the total composition,

methionine, in an amount of from 0.1 to 3% by weight, calculated on the weight of the total composition, or cysteine, in an amount of at most 0.009% by weight, calculated on the weight of the total composition, or a derivative of cysteine, obtained by acylation of the aminogroup or acylation of the sulphydrylgroup such as N-acetyl cysteine or S-acetylcysteine in an amount of from 0.1—3% and preferably 1—2% by weight, optionally formed in situ from means providing such compounds due to spontaneous conversion,

carraghenate, in an amount of from 0.25 to 4% by weight, calculated on the weight of the total composition,

sodium chloride, in an amount of from 0.1 to 5% by weight, calculated on the weight of the total composition,

a preservative, in an amount of from 0.1 to 2% by weight, calculated on the weight of the total composition,

water, ad 100% by weight, calculated on the weight of the total composition.

2. Process according to Claim 1, characterized in that in the aqueous phase panthenol, methionine and/or cystine or a derivative of cysteine, sodium chloride and a preservative are dissolved, followed by addition and dissolution of the carraghenate.

3. Process according to Claim 1, characterized in that panthenol is added in an amount of from 1 to 2% by weight, calculated on the weight of the total composition.

4. Process according to Claim 1, characterized in that it contains at least methionine in an amount of from 1 to 2% by weight, calculated on the weight of the total composition.

5. Process according to Claim 1, characterized in that it contains at least carraghenate in an amount of from 1 to 2% by weight, calculated on the weight of the total composition.

6. Process according to Claim 1, characterized in that it contains at least sodium chloride in an amount of 0.9% by weight, calculated on the weight of the total composition.

7. Process according to Claim 1, characterized in that it contains as preservative a methyl- and/or propyl ester of p-hydroxybenzoic acid, optionally mixed with phenylglycol.

8. Process according to Claim 7, characterized in that it contains methyl-p-hydroxybenzoate in an amount of 0.2% by weight, calculated on the weight of the total composition.

9. Process according to Claim 7, characterized in that it contains propyl-p-hydroxybenzoate in an amount of 0.1% by weight, calculated on the weight of the total composition.

10. Process according to Claim 1, characterized in that it contains perfume in an amount of from 0.1 to 0.5% by weight, calculated on the weight of the total composition and/or antioxydants in an amount of from 0.01 to 0.3% by weight, calculated on the weight of the total composition.

**Patentansprüche für die Vertragsstaaten: BE CH DE FR GB IT LI LU NL SE.**

1. Zusammensetzung für die Behandlung eines Sonnenekzems oder von Dishydrosis, dadurch gekennzeichnet, daß sie wenigstens aus:

Panthenol, in einer Menge von 0,1 bis 5 Gew.-%, bezogen auf das Gewicht der gesamten Zusammensetzung,

Methionin, in einer Menge von 0,1 is 3 Gew.-%, bezogen auf das Gewicht der gesamten Zusammensetzung, oder Cystin, in einer Menge von höchstens 0,009 Gew.-%, bezogen auf das Gewicht der gesamten Zusammensetzung, oder einem Cysteinderivat, wie N-Acetylcystein oder S-Acetylcystein, das durch Acylierung der Aminogruppe oder durch Acylierung der Sulfhydrylgruppe erhalten wurde, in einer Menge von 0,1 bis 3 Gew.-% und vorzugsweise von 1 bis 2 Gew.-%, das gegebenenfalls in situ durch Mittel gebildet wurde, die solche Verbindungen durch spontane Umwandlungen zur Verfügung stellen,

Carraghenat, in einer Menge von 0,25 bis 4 Gew.-%, bezogen auf das Gewicht der gesamten Zusammensetzung,

Natriumchlorid, in einer Menge von 0,1 bis 5 Gew.-%, bezogen auf das Gewicht der gesamten Zusammensetzung,

einem Konservierungsmittel, in einer Menge von 0,1 bis 2 Gew.-%, bezogen auf das Gewicht der gesamten Zusammensetzung, sowie

Wasser ad 100 Gew.-%, bezogen auf das Gewicht der gesamten Zusammensetzung, besteht.

2. Zusammensetzung nach Anspruch 1, dadurch gekennzeichnet, daß sie wenigstens Panthenol in einer Menge von 1 bis 2 Gew.-%, bezogen auf das Gewicht der gesamten Zusammensetzung, enthält.

3. Zusammensetzung nach Anspruch 1, dadurch gekennzeichnet, daß sie wenigstens Methionin in einer Menge von 1 bis 2 Gew.-%, bezogen auf das Gewicht der gesamten Zusammenzetzung, enthält.

4. Zusammensetzung nach Anspruch 1, dadurch gekennzeichnet, daß sie wenigstens Carraghenat in einer Menge von 1 bis 2 Gew.-%, bezogen auf das Gewicht der gesamten Zusammensetzung, enthält.

5. Zusammensetzung nach Anspruch 1, dadurch gekennzeichnet, daß sie wenigstens Natriumchlorid in einer Menge von 0,9 Gew.-%, bezogen auf das Gewicht der gesamten Zusammensetzung, enthält.

6. Zusammensetzung nach Anspruch 1, dadurch gekennzeichnet, daß sie als Konservierungsmittel einen Methyl- und/oder Propylester der p-Hydroxybenzoesäure, gegebenenfalls gemischt mit Phenylglykol, enthält.

7. Zusammensetzung nach Anspruch 6, dadurch gekennzeichnet, daß sie Methyl-p-hydroxybenzoat in einer Menge von 0,2 Gew.-%, bezogen auf das Gewicht der gesamten Zusammensetzung, enthält.

8. Zusammensetzung nach Anspruch 6, dadurch gekennzeichnet, daß sie Propyl-p-hydroxybenzoat in einer Menge von 0,1 Gew.-%, bezogen auf das Gewicht der gesamten Zusammensetzung, enthält.

9. Zusammensetzung nach Anspruch 1, dadurch gekennzeichnet, daß sie einen Duftstoff in einer Menge von 0,1 bis 0,5 Gew.-%, bezogen auf das Gewicht der gesamten Zusammensetzung, und/oder Antioxidationsmittel in einer Menge von 0,01 bis 0,3 Gew.-%, bezogen auf das Gewicht der gesamten Zusammensetzung, enthält.

**Patentansprüche für den Vertragsstaat: AT**

1. Verfahren zur Herstellung einer Zusammensetzung zur Behandlung eines Sonnenekzems oder von Dishydrosis, dadurch gekennzeichnet, daß die Zusammenzetzung durch Zusammenmischen wenigstens folgender Bestandteile hergestellt wird:

Panthenol, in einer Menge von 0,1 bis 5 Gew.-%, bezogen auf das Gewicht der gesamten Zusammensetzung,

Methionin, in einer Menge von 0,1 bis 3 Gew.-%, bezogen auf das Gewicht der gesamten Zusammensetzung, oder Cystin, in einer Menge von höchstens 0,009 Gew.-%, bezogen auf das Gewicht der gesamten Zusammensetzung, oder eines Cysteinderivats, wie N-Acetylcystein oder S-Acetylcystein, das durch Acylierung der Aminogruppe oder durch Acylierung der Sulfhydrylgruppe erhalten wurde, in einer Menge von 0,1 bis 3 Gew.-% und vorzugsweise von 1 bis 2 Gew.-%, das gegebenenfalls in situ durch Mittel gebildet wurde, die solche Verbindungen durch spontane Umwandlungen zur Verfügung stellen,

Carraghenat in einer Menge von 0,25 bis 4 Gew.-%, bezogen auf das Gewicht der gesamten Zusammensetzung,

Natriumchlorid, in einer Menge von 0,1 bis 5 Gew.-%, bezogen auf das Gewicht der gesamten Zusammensetzung,

eines Konservierungsmittels, in einer Menge von 0,1 bis 2 Gew.-%, bezogen auf das Gewicht der gesamten Zusammensetzung, sowie

Wasser ad 100 Gew.-%, bezogen auf das Gewicht der gesamten Zusammensetzung.

2. Verfahren nach Anspruch 1, dadurch gekennzeichnet, daß in der wäßrigen Phase Panthenol, Methionin und/oder Cystin oder ein Cysteinderivat, Natriumchlorid und ein Konservierungsmittel gelöst werden, woraufhin die Zugabe und Auflösung des Carraghenats erfolgt.

3. Verfahren nach Anspruch 1, dadurch gekennzeichnet, daß Panthenol in einer Menge von 1 bis 2 Gew.-%, bezogen auf das Gewicht der gesamten Zusammensetzung, zugegeben wird.

4. Verfahren nach Anspruch 1, dadurch gekennzeichnet, daß wenigstens Methionin in einer Menge von 1 bis 2 Gew.-%, bezogen auf das Gewicht der gesamten Zusammensetzung, zugegeben wird.

5. Verfahren nach Anspruch 1, dadurch gekennzeichnet, daß wenigstens Carraghenat in einer Menge von 1 bis 2 Gew.-%, bezogen auf das Gewicht der gesamten Zusammensetzung, zugegeben wird.

6. Verfahren nach Anspruch 1, dadurch gekennzeichnet, daß wenigstens Natriumchlorid in einer Menge von 0,9 Gew.-%, bezogen auf das Gewicht der gesamten Zusammensetzung, zugegeben wird.

7. Verfahren nach Anspruch 1, dadurch gekennzeichnet, daß als Konservierungsmittel ein Methyl- und/oder Propylester der p-Hydroxybenzoesäure zugegeben wird, gegebenenfalls gemischt mit Phenylglykol.

8. Verfahren nach Anspruch 7, dadurch gekennzeichnet, daß Methyl-p-hydroxybenzoat in einer Menge von 0,2 Gew.-%, bezogen auf das Gewicht der gesamten Zusammensetzung, zugegeben wird.

9. Verfahren nach Anspruch 7, dadurch gekennzeichnet, daß Propyl-p-hydroxybenzoat in einer Menge von 0,1 Gew.-%, bezogen auf das Gewicht der gesamten Zusammensetzung, zugegeben wird.

10. Verfahren nach Anspruch 1, dadurch gekennzeichnet, daß ein Duftstoff in einer Menge von 0,1 bis 0,5 Gew.-%, bezogen auf das Gewicht der gesamten Zusammensetzung, und/oder Antioxidationsmittel in einer Menge von 0,01 bis 0,3 Gew.-%, bezogen auf das Gewicht der gesamten Zusammensetzung, zugegeben werden.

**Revendications pour les Etats contractants: BE CH DE FR GB IT LI LU NL SE**

1. Composition pour le traitement de l'eczéma solaire ou dysidrose, caractérisée en ce qu'elle contient au moins les ingrédients suivants:
panthénol en une quantité de 0,1 à 5% en poids, sur la base du poids de la composition totale,
méthionine, en une quantité de 0,1 à 3% en poids, sur la base de la composition totale, ou cystine en une quantité d'au plus 0,009% en poids sur la base du poids de la composition totale, ou un dérivé de cystéine obtenu par acylation du groupe amino- ou acylation du groupe sulfhydryle tel que N-acétylcystéine ou S-acétylcystéine en une quantité de 0,1 à 3% et de préférence 1 à 2% en poids, facultativement formée in situ par des moyens procurant ces composés par conversion spontanée,
carraghénate, en une quantité de 0,25 à 4% en poids, sur la base du poids de la composition totale,
chlorure de sodium, en une quantité de 0,1 à 5% en poids, sur la base du poids de la composition totale,
un conservateur, en une quantité de 0,1 à 2% en poids, sur la base du poids de la composition totale,
eau, le complément à 100% en poids, calculé sur le poids de la composition totale.

2. Composition selon la revendication 1, caractérisée en ce qu'elle contient au moins du panthénol en une quantité de 1 à 2% en poids, sur la base du poids de la composition totale.

3. Composition selon la revendication 1, caractérisée en ce qu'elle contient au moins de la méthionine en une quantité de 1 à 2% en poids, calculée sur le poids de la composition totale.

4. Composition selon la revendication 1, caractérisée en ce qu'elle contient au moins du carraghénate en une quantité de 1 à 2%, sur la base du poids de la composition totale.

5. Composition selon la revendication 1, caractérisée en ce qu'elle contient au moins du chlorure de sodium en une quantité de 0,9% en poids, sur la base du poids de la composition totale.

6. Composition selon la revendication 1, caractérisée en ce qu'elle contient comme conservateur un ester méthylique et/ou propylique de l'acide p-hydroxybenzoïque, facultativement mélangé avec du phényl glycol.

7. Composition selon la revendication 6, caractérisée en ce qu'elle contient du p-hydroxybenzoate de méthyle en une quantité de 0,2% en poids, sur la base du poids de la composition totale.

8. Composition selon la revendication 6, caractérisée en ce qu'elle contient du p-hydroxybenzoate de propyle en une quantité de 0,1% en poids, sur la base du poids de la composition totale.

9. Composition selon la revendication 1, caractérisée en ce qu'elle contient un parfum en une quantité de 0,1 à 0,5% en poids, sur la base du poids de la composition totale, et/ou des antioxydants en une quantité de 0,01 à 0,3% en poids, sur la base du poids de la composition totale.

**Revendications pour l'Etat contractant: AT**

1. Procédé pour la préparation d'une composition pour le traitement de l'eczéma solaire ou dysidrose, caractérisé en ce qu'on prépare une composition en mélangeant ensemble au moins les ingrédients suivants:
panthénol, en une quantité de 0,1 à 5% en poids, sur la base du poids de la composition totale,
méthionine, en une quantité de 0,1 à 3% en poids, sur la base du poids de la composition totale, ou cystine, en une quantité d'au plus 0,009% en poids, sur la base du poids de la composition totale, ou un dérivé de cystéine, obtenu par acylation du groupe amino- ou acylation du groupe sulfhydryle, tel que N-acétylcystéine ou S-acétylcystéine, en une quantité de 0,1 à 3%, et de préférence 1 à 2% en poids, facultativement formée in situ à partir de moyens procurant ces composés par conversion spontanée,

carraghénate, en une quantité de 0,25 à 4% en poids, sur la base du poids de la composition totale,

chlorure de sodium, en une quantité de 0,1 à 5% en poids, sur la base du poids de la composition totale,

un conservateur, en une quantité de 0,1 à 2% en poids, sur la base du poids de la composition totale,

eau, complément à 100% en poids, sur la base du poids de la composition totale.

2. Procédé selon la revendication 1, caractérisé en ce qu'on dissout dans la phase aqueuse le panthénol, la méthionine et/ou la cystine ou un dérivé de cystéine, le chlorure de sodium et un conservateur, après quoi on ajoute et on dissout le carraghénate.

3. Procédé selon la revendication 1, caractérisé en ce qu'on ajoute le panthénol en une quantité de 1 à 2% en poids, sur la base du poids de la composition totale.

4. Procédé selon la revendication 1, caractérisé en ce que la composition contient au moins la méthionine en une quantité de 1 à 2% en poids, sur la base du poids de la composition totale.

5. Procédé selon la revendication 1, caractérisé en ce que la composition contient au moins le carraghénate en une quantité de 1 à 2% en poids, sur la base du poids de la composition totale.

6. Procédé selon la revendication 1, caractérisé en ce que la composition contient au moins le chlorure de sodium en une quantité de 0,9% en poids, sur la base du poids de la composition totale.

7. Procédé selon la revendication 1, caractérisé en ce que la composition contient comme conservateur un ester méthylique et/ou propylique de l'acide p-hydroxybenzoïque, facultativement mélangé avec de phényl glycol.

8. Procédé selon la revendication 7, caractérisé en ce que la composition contient le p-hydroxybenzoate de méthyle en une quantité de 0,2% en poids, sur la base du poids de la composition totale.

9. Procédé selon la revendication 7, caractérisé en ce que la composition contient le p-hydroxybenzoate de propyle en une quantité de 0,1% en poids, sur la base du poids de la composition totale.

10. Procédé selon la revendication 1, caractérisé en ce que la composition contient du parfum en une quantité de 0,1 à 0,5% en poids, sur la base du poids de la composition totale, et/ou des antioxydants en une quantité de 0,01 à 0,3% en poids, sur la base du poids de la composition totale.